# EUROPEAN PATENT APPLICATION

(11) **EP 1 362 863 A1**
(43) Date of publication of application: **19.11.2003**
(21) Application number: 02712407.2
(22) Date of filing: 18.02.2002
(51) Int. Cl.: C07K 1/06, C07K 1/113, C07K 16/00

(54) **MODIFIER**

(30) Priority: 20.02.2001 JP 2001043942
(71) Applicant: TAKARA BIO INC., Otsu-shi, Shiga 520-2193 (JP)
(72) Inventor: ENOKI, Tatsuji, Otsu-shi, Shiga 520-0865 (JP); SAGAWA, Hiroaki, Kusatsu-shi, Shiga 525-0025 (JP); SAKAI, Takeshi, wamachi,Hirosaki-shi, Aomori 036-8183 (JP); KATO, Ikunoshin, Uji-shi, Kyoto 611-0028 (JP)
(74) Representative: Vossius, Volker, Dr.
(86) International application number: JP0201336
(87) International publication number: WO02066496

(57) **Abstract**

A modifier characterized by containing as the active ingredient a compound represented by the formula (I) (wherein X is OH, OSO₃H, or OCH₃ and R is a substituent excluding OH). The modifier is applied to a substance having reactivity with the compound.

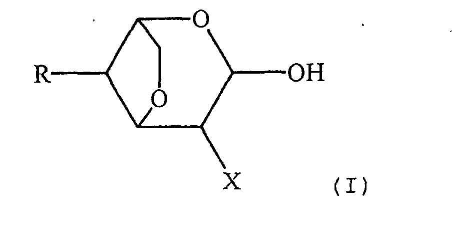

## Description

### Technical Field

The present invention relates to a composition for modifying a physiologically active substance such as a peptide, a kit for modification, a modification product obtained using the same, and a method for modification. A physiologically active substance having an improved property and the like can be provided according to the present invention.

### Background Art

Oligopeptides and polypeptides have their inherent amino acid sequences, molecular weights and conformations defined by the amino acid sequences. Many of them are physiologically active substances each having inherent physiological function.

It is very difficult to isolate a substance such as a polypeptide present in a trace amount in an organism. Recombinant DNA techniques have enabled preparation of such a substance in large quantities using a microorganism or the like.

Establishment of hybridoma techniques has enabled supply of a monoclonal antibody in large quantities and provided a highly sensitive immunological assay for an antigen.

A polypeptide produced using recombinant DNA techniques often exists as a denatured insoluble substance in a host cell. Solubilization of such an insoluble polypeptide has been desired.

Also, a monoclonal antibody obtained using hybridoma techniques sometimes exhibits a water-insoluble property. Improvement of the water solubility has been desired in many cases.

Many kinds of chemically modifying agents are used for improving the property of a physiologically active substance. The function of the physiologically active substance of which the modification is required may be spoiled depending of the chemical modification reaction conditions. Provision of a more convenient method for modifying a target substance of which the modification is required (e.g., a physiologically active substance) under milder conditions has been desired.

### Objects of Invention

The main object of the present invention is to provide a modifying composition that can be used to conveniently modify a target substance of which the modification is required (e.g., an oligopeptide or a polypeptide) under mild conditions, a kit for modification, a modification product of a target substance of which the property is improved using the same, and a method for modifying a target substance.

### Summary of Invention

The present invention is outlined as follows. The first aspect of the present invention relates to a composition for modifying a target substance which contains a compound represented by formula I as an active ingredient, wherein the target substance is reactive with said compound: 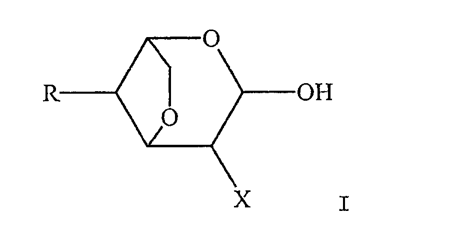 wherein X is OH, OSO₃H or OCH₃; and R is a substituent other than OH.

The second aspect of the present invention relates to a kit for modifying a target substance which contains a compound represented by formula I, wherein the target substance is reactive with said compound.

The third aspect of the present invention relates to a modification product of a target substance, which is obtained by reacting a target substance with a compound represented by formula I, wherein the target substance is reactive with said compound.

The fourth aspect of the present invention relates to a method for modifying a target substance, the method comprising reacting a target substance with a compound represented by formula I, wherein the target substance is reactive with said compound.

The fifth aspect of the present invention relates to a mixture of a compound represented by formula I and a target substance that is reactive with said compound.

In one embodiment of the first to fifth aspect, R in formula I is a sugar. The compound represented by formula I is exemplified by a saccharide having 3,6-anhydrogalactose at the reducing end. The saccharide is exemplified by a saccharide selected from the group consisting of agarobiose, agarotetraose, agarohexaose and agarooctaose (these substances may be generically referred to as agarooligosaccharides hereinafter), as well as carrabiose, carratetraose, carrahexaose and carraoctaose (these substances may be generically referred to as carraoligosaccharides hereinafter).

In one embodiment of the first to fifth aspect, the target substance is a peptide. The peptide is exemplified by a physiologically active substance selected from an antibody, an enzyme, a hormone or a cytokine.

In one embodiment of the second aspect, the kit may contain a reducing agent.

In one embodiment of the fourth aspect, the target substance may be reacted with the compound represented by formula I under neutral to acidic conditions, for 10 seconds to 72 hours, at 5 to 45°C.

As used herein, the term "peptide" encompasses an oligopeptide and a polypeptide. A peptide consisting of ten amino acid residues or less is defined as an oligopeptide. A peptide consisting of eleven amino acid residues or more is defined as a polypeptide. Polypeptides include proteins. According to the present invention, a sugar is, for example, a monosaccharide, an oligosaccharide or a polysaccharide. A sugar consisting of ten constituting saccharides or less is defined as an oligosaccharide. A sugar consisting of eleven constituting saccharides or more is defined as a polysaccharide.

In one embodiment of the present invention, an antibody that contains galactose and of which the water solubility is improved is provided by modifying an antibody as a target substance using a modifying composition containing an agarooligosaccharide as an active ingredient.

### Detailed Description of the Invention

Hereinafter, the present invention will be explained in detail.

There is no specific limitation concerning "R" in the compound used according to the present invention as long as the compound can be used to alter a property of a target substance. For example, it is a sugar, a labeled compound or a substance having a tissue-specific affinity.

The compound represented by formula I used according to the present invention is exemplified by a compound having 3,6-anhydrogalactopyranose, or a sulfated or methylated derivative thereof, at the reducing end. Examples of such compounds include agarooligosaccharides and carraoligosaccharides such as agarobiose, agarotetraose, agarohexaose, agarooctaose, carrabiose, carratetraose, carrahexaose and carraoctaose. A target substance of interest (e.g., a peptide) can be conveniently modified with galactose or the like using a compound selected from the above-mentioned compounds. A peptide can be modified with such an oligosaccharide by a reaction for 10 seconds to 72 hours at 5 to 45°C in a buffer at neutral to acidic pH containing the oligosaccharide at a concentration of 0.1-100 mg/ml with the ratio of the oligosaccharide : the peptide = 100-1 : 1. As a result of the reaction, the water solubility of the peptide modified with galactose or the like is remarkably improved.

There is no specific limitation concerning the reaction conditions used for the step of reacting a target substance that is reactive with a compound represented by formula I of the present invention with said compound as long as a modification product is efficiently obtained and the physiological function or the like inherent to the target substance is not spoiled.

For example, if a peptide is to be modified using a compound selected from the above-mentioned oligosaccharides, the modification product of the present invention can be obtained, preferably, by a reaction for 10 seconds to 72 hours at 5 to 45°C in a buffer at neutral to acidic pH containing the oligosaccharide at a concentration of 0.1-100 mM with the ratio of the oligosaccharide : the target substance = 100-1 : 1.

Thus, according to the present invention, a target substance that is reactive with a compound represented by formula I is a substance that is modified with R of the compound represented by formula I by a reaction for 10 seconds to 72 hours at 5 to 45 °C in a buffer at neutral to acidic pH containing the compound at a concentration of 0.1-100 mM with the ratio of the compound : the target substance = 100-1 : 1. It is possible to determine whether or not the substance is modified with R by analyzing physical and chemical properties of a product obtained after the reaction according to conventional methods. For example, if a peptide is modified with galactose by reacting the peptide with agarobiose, the presence of modification can be confirmed by subjecting the modification product to electrophoresis and comparing the mobilities of the unmodified substance with the modification product. Alternatively, galactose contained in a decomposition product obtained by hydrolysis of the modification product may be analyzed. For example, if an amino acid is used as a target substance, a structure of a product obtained after a reaction with agarobiose may be analyzed.

According to the present invention, if a physiologically active substance is used as a target substance, it is preferable to determine the conditions such that a compound represented by formula I does not react at the physiologically active site. For example, if an antibody is used as a target substance, a reaction with a compound represented by formula I may be carried out after binding the antibody to its antigen. If an enzyme is used as a target substance, a reaction with a compound represented by formula I may be carried out after binding the enzyme to its substrate.

A more stable modification product can be obtained by optionally subjecting the modification product of the present invention to reduction. According to the present invention, reduction may be carried out using a conventional reduction method. Although it is not intended to limit the present invention, for example, reduction may be carried out according to a conventional method using a reagent such as dimethylamine-borane complex, pyridylamine-borane complex, trimethylamine-borane complex, sodium cyanotrihydroborate or sodium boron hydride as a reducing agent.

The modifying composition of the present invention may be formulated according to a conventional method after mixing a compound represented by formula I with a known liquid or solid carrier.

The kit for modification of the present invention contains a compound represented by formula I. It may optionally contain one or more selected from a reducing agent, a control substance, a buffer or the like.

A product modified with R can be obtained conveniently under mild conditions by reacting a target substance with a compound represented by formula I. The modification product in the reaction mixture may optionally be purified by a conventional purification method and used as a purified modification product.

For example, an antibody modified with a galactose-containing compound can be obtained by using a compound represented by formula I (e.g., an agarooligosaccharide) to conveniently modify a target substance (e.g., a peptide such as a monoclonal antibody) with a galactose-containing compound.

It is conventionally known that an antigen-binding ability of an antibody may be abolished upon modification of the antibody depending on the reaction conditions. An antibody with such modification tends to deposit during storage and be insolubilized. On the other hand, the present invention enables modification of an antibody (e.g., a monoclonal antibody) under mild conditions. The modification product obtained according to the present invention has remarkably improved properties as follows: the antigen-binding ability is not spoiled; the water-solubility is remarkably improved; and the insolubilization during storage is prevented.

A target substance can be modified in vivo with R by administering a compound represented by formula I as an active ingredient to a living body. The dosage form may be for oral administration or for injection, and may be prepared according to a conventional method using the compound represented by formula I as an active ingredient. The administration may be carried out orally, intravenously or intramuscularly. The dosage of the compound represented by formula I is generally 10 µg to 200 mg/kg although it varies depending on the target substance to be modified in vivo.

The target substance in a living body is exemplified by an antibody or an enzyme. The present invention is useful for treatment or prevention of a disease that requires in vivo modification of a target substance for the treatment or prevention (e.g., rheumatism).

No acute toxicity is observed when a compound represented by formula I such as an agarooligosaccharide is administered to a mouse at a dosage of 1 g/kg.

The present invention provides a composition for adding R to a target substance which contains a compound represented by formula I as an active ingredient. The present invention also provides a method for adding R to a target substance using a compound represented by formula I as an active ingredient.

Furthermore, the present invention provides use of a compound represented by formula I as an agent for modifying a target substance with R. In addition, the present invention provides a method for treating a disease that requires modification of a target substance with R for the treatment using a compound represented by formula I as an active ingredient.

### Examples

The following Examples further illustrate the present invention in detail but are not to be construed to limit the scope thereof.

### Example 1

(1) Commercially available agar (Ina agar type S-7, Ina Shokuhin Kogyo) was dissolved in deionized water at a concentration of 10%(w/v). A strong cation exchange resin (Diaion, Mitsubishi Chemical, SK-104) converted into hydrogen ion type was further added thereto at a concentration of 1%(w/v). After reacting at 90°C for 3 hours, the reaction mixture was cooled to normal temperature and subjected to filtration to remove the resin. The filtrate was treated with active carbon at a concentration of 2%(w/v) to remove colored substances and the like, and filtrated through a filter with pore size of 1 µm. After neutralization, the filtrate was lyophilized according to a conventional method to prepare agarooligosaccharides.
   The agarooligosaccharides contained water (2.3%), galactose (9.8%), agarobiose (44.1%) as well as agarotetraose, agarohexaose, agarooctaose and the like (43.4%). The pH of an aqueous solution containing the agarooligosaccharides at a concentration of 1% was 4.0.
   The agarooligosaccharides were subjected to normal phase HPLC under the conditions as described below to separate agarobiose, agarotetraose, agarohexaose and agarooctaose:
   Column: TSK-gel Amide-80 (21.5 mm x 300 mm, Tosoh);
   Solvent A: 90% acetonitrile aqueous solution;
   Solvent B: 50% acetonitrile aqueous solution;
   Flow rate: 5 ml/min;
   Elution: linear gradient from Solvent A to Solvent B (80 minutes) → Solvent B (20 minutes); and
   Detection: absorbance at 195 nm.
(2) Carraoligosaccharides were prepared according to the method as described in Example 1-(1) using commercially available κ-carrageenan (Sigma). Carrabiose, carratetraose, carrahexaose and carraoctaose were separated from the carraoligosaccharides.

### Example 2

A solution containing the agarooligosaccharides as described in Example 1-(1) at a concentration of 100 mg/ml and a solution containing bovine serum albumin (Sigma, A0281) at a concentration of 11.1 mg/ml in PBS (pH 7.2) were mixed at a ratio of 1 : 9. The mixture was reacted at 37°C for 24 hours to obtain a modification product of bovine serum albumin.

Similarly, a solution containing the agarooligosaccharides as described in Example 1-(1) at a concentration of 100 mg/ml and a solution containing human immunoglobulin G (Oriental Yeast, IgM-04) at a concentration of 11.1 mg/ml in PBS were mixed at a ratio of 1 : 9. The mixture was reacted at 37°C for 24 hours to obtain a modification product of human immunoglobulin G.

The modification products of bovine serum albumin and human immunoglobulin G were subjected to electrophoresis on SDS-polyacrylamide gel together with unmodified counterparts. Shifts to higher molecular weights as compared with the unmodified counterparts were observed for both of the modification products. Thus, it was strongly suggested that the agarooligosaccharides were attached to bovine serum albumin and human immunoglobulin G.

In order to confirm that the agarooligosaccharides were attached to the proteins, the modification products of the proteins were dialyzed to remove free agarooligosaccharides, and only high molecular weight fractions were collected by HPLC. HPLC was carried out as follows.

Column: Shodex SB-2003 (20 mm x 300 mm, Showa Denko);
Solvent: 0.1 M NaCl;
Flow rate: 3 ml/min; and
Fraction: 1.5 ml/tube.

The protein concentrations in the separated fractions were determined by measuring the absorbances at 280 nm, and fractions containing the proteins were collected. As a control, unmodified counterparts for both of the proteins were also separated under the same conditions. Next, the galactose contents in the collected protein fractions were measured. Specifically, the protein fractions were adequately dialyzed, subjected to hydrolysis in 2 N HCl at 100°C for three hours, and then dried. The reducing ends of the resulting monosaccharides were fluorescence-labeled with 2-aminopyridine using GlycoTAG and GlycoTAG Reagent kit (both from Takara Shuzo). The sugar compositions were analyzed according to a conventional method by HPLC (Agric. Biol. Chem. 55, 283-284 (1991)) to determine the galactose contents in the respective protein fractions. Then, comparisons between the modification products and the unmodified counterparts were carried out.

As a result, it was shown that eight molecules of galactose were attached to one molecule of the modification product of bovine serum albumin, whereas three or four molecules of galactose were attached to one molecule of the modification product of human immunoglobulin G.

Furthermore, it was confirmed that galactose residues were introduced into proteins when agarobiose, agarotetraose, agaropentaose, agarooctaose, carrabiose, carratetraose, carrapentaose and carraoctaose separated in Example 1-(1) and (2) were used for similar treatments.

### Example 3

The modification products of bovine serum albumin and human immunoglobulin G separated using HPLC as described in Example 2 were adequately dialyzed against water and lyophilized. The lyophilized modification products and lyophilized unmodified counterparts for both of the proteins were similarly dissolved in water or PBS. The solubility of the modification products in each solvent was superior to that of the unmodified counterparts.

### Example 4

A solution of the agarooligosaccharides prepared in Example 1-(1) in saline (Otsuka Pharmaceutical) was intravenously administered to three male SD rats (Charles River, 9 weeks old) at a dosage of 60 mg/kg once a day for five successive day.

Sera were prepared from the rats five hours after the final administration. Immunoglobulin G was separated from the sera using a protein A column (Pharmacia Hitrap Protein A, Pharmacia). Immunoglobulin G was also prepared in a similar manner from mice in a group without the agarooligosaccharide administration. The contents of galactose attached to immunoglobulin G from the group with or without the administration were compared. Galactose was quantified as described in Example 2.

As a result, one extra molecule of galactose per 2.5 molecules of immunoglobulin was detected for the group with the administration as compared with the group without the administration.

### Industrial Applicability

The present invention provides a modifying composition that can be used to conveniently modify a target substance under conditions milder than conventional ones. The modifying composition is useful for alteration of a physical property (e.g., improvement of water solubility) of a target substance. The present invention also provides a target substance modified using said modifying composition. Such a target substance with modification is useful as a highly functional target substance having an additional function.

Furthermore, the modifying composition of the present invention enables in vivo modification of a target substance. Thus, it is useful for a composition for treating or preventing a disease that requires in vivo modification of a target substance for the treatment or prevention.

## Claims

1. A composition for modifying a target substance which contains a compound represented by formula I as an active ingredient, wherein the target substance is reactive with said compound: 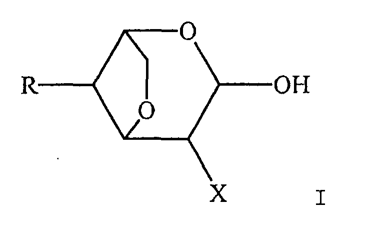 wherein X is OH, OSO₃H or OCH₃; and R is a substituent other than OH.

2. The composition according to claim 1, wherein R in formula I is a sugar.

3. The composition according to claim 1, wherein the target substance is a peptide.

4. The composition according to claim 3, wherein the peptide is an oligopeptide or a polypeptide.

5. A kit for modifying a target substance which contains a compound represented by formula I, wherein the target substance is reactive with said compound.

6. The kit according to claim 5, wherein R in formula I is a sugar.

7. The kit according to claim 5, wherein the target substance is a peptide.

8. The kit according to claim 7, wherein the peptide is an oligopeptide or a polypeptide.

9. The kit according to claim 5, which contains a reducing agent.

10. A modification product of a target substance, which is obtained by reacting a target substance with a compound represented by formula I, wherein the target substance is reactive with said compound.

11. The product according to claim 10, wherein the target substance which has been reacted with the compound represented by formula I is further reacted with a reducing agent.

12. The product according to claim 10, wherein R in formula I is a sugar.

13. The product according to claim 10, wherein the target substance is a peptide.

14. The product according to claim 13, wherein the peptide is an oligopeptide or a polypeptide.

15. The product according to claim 13, wherein the peptide is an antibody.

16. A method for modifying a target substance, the method comprising reacting a target substance with a compound represented by formula I, wherein the target substance is reactive with said compound.

17. The method according to claim 16, wherein R in formula I is a sugar.

18. The method according to claim 17, wherein the compound represented by formula I is a saccharide having 3,6-anhydrogalactose at the reducing end.

19. The method according to claim 18, wherein the saccharide is selected from the group consisting of agarobiose, agarotetraose, agarohexaose, agarooctaose, carrabiose, carratetraose, carrahexaose and carraoctaose.

20. The method according to claim 16, wherein the target substance is a peptide.

21. The method according to claim 20, wherein the peptide is an oligopeptide or a polypeptide.

22. The method according to claim 20, wherein the peptide is an antibody.

23. The method according to claim 16, wherein the target substance is reacted with the compound represented by formula I under neutral to acidic conditions, for 10 seconds to 72 hours, at 5 to 45°C.
